**Europäisches Patentamt**

**European Patent Office**

**Office européen des brevets**

(19)

(11) Numéro de publication : **0 364 507 B1**

(12)

# FASCICULE DE BREVET EUROPEEN

(45) Date de publication du fascicule du brevet :
**08.07.92 Bulletin 92/28**

(51) Int. Cl.$^5$ : **A61F 2/08**

(21) Numéro de dépôt : **89900839.5**

(22) Date de dépôt : **22.12.88**

(86) Numéro de dépôt international :
**PCT/FR88/00634**

(87) Numéro de publication internationale :
**WO 89/05614 29.06.89 Gazette 89/14**

(54) **LIGAMENT SYNTHETIQUE POUR GENOU.**

(30) Priorité : **22.12.87 FR 8718247**

(43) Date de publication de la demande :
**25.04.90 Bulletin 90/17**

(45) Mention de la délivrance du brevet :
**08.07.92 Bulletin 92/28**

(84) Etats contractants désignés :
**BE DE FR GB IT**

(56) Documents cités :
**EP-A- 0 236 821**
**EP-A- 0 239 775**
**EP-A- 0 249 346**
**FR-A- 2 135 825**
**FR-A- 2 213 761**
**FR-A- 2 596 641**
**FR-A- 2 598 311**
**FR-A- 2 598 315**
**US-A- 4 411 027**

(73) Titulaire : **RHENTER, Jean Luc**
**11, rue de l'Annonciade**
**F-69001 Lyon (FR)**

(72) Inventeur : **RHENTER, Jean-Luc**
**11, rue de l'Annonciade**
**F-69001 Lyon (FR)**
Inventeur : **COLLOMB, Jean**
**L'Olagnier**
**F-26800 Portes-lès-Valence (FR)**

(74) Mandataire : **Laurent, Michel et al**
**Cabinet LAURENT et CHARRAS, 20, rue Louis**
**Chirpaz B.P. 32**
**F-69131 Ecully Cedex (FR)**

EP 0 364 507 B1

Jouve, 18, rue Saint-Denis, 75001 PARIS

## Description

La présente invention concerne un nouveau type de ligament synthétique, notamment destiné à remplacer le ligament croisé antérieur du genou selon le préambule de la revendication 1. Un tel ligament est connu du document EP-A-0249346 (voir Figures 4,5,6).

Le ligament croisé antérieur du genou unit l'échancrure interglénoïdienne du tibia à l'échancrure intercondylienne du fémur. Son rôle principal consiste à freiner le glissement du tibia vers l'avant, c'est-à-dire à réduire le mouvement de tiroir antérieur.

La chirurgie orthopédique du genou a largement évolué ces dernières années et on tend de plus en plus à proposer des prothèses de remplacement des ligaments.

Les prothèses de remplacement du ligament croisé antérieur dans le genou proposées jusqu'alors sont constituées d'un seul faisceau de fibres, ou de groupes de fibres synthétiques, se terminant toutes en un seul et même lieu à chacune des extrémités dudit faisceau. Cette prothèse permet de remplacer le ligament croisé antérieur et de limiter le mouvement de tiroir antérieur du tibia sous le fémur créé par la liaison du ligament à remplacer.

Toutefois, ce système à un seul faisceau de fibres ou de groupes de fibres ne permet de limiter ce mouvement de tiroir qu'à certains degrés de flexion. En effet, un seul faisceau de fibres ne permet pas une tension isométrique du ligament synthétique à chaque degré de flexion, c'est-à-dire une tension sans raccourcissement du ligament. Un tel ligament est décrit dans l'un des exemples d'application d'un instrument chirurgical destiné à la mise en place sur les articulations de tels ligaments dans le document FR - A - 2 598 311. Ce ligament est mono-brin, et décrit, lorsqu'il est en place dans l'articulation, un simple aller-retour à partir de la face antéro-interne du tibia autour d'un point d'ancrage ménagé au niveau du fémur. De la sorte, du fait de l'indépendance du brin aller par rapport au brin retour, il n'y a jamais report des contraintes d'un brin sur l'autre, ce qui diminue la résistance du brin sollicité, et donc sa durée de vie. De plus, tel qu'il est décrit, ce ligament n'empêche nullement les mouvements de tiroir antérieur du tibia sous le fémur.

De fait, la non possibilité pour un ligament mono-brin de limiter le mouvement de tiroir antérieur du tibia sous le fémur est inhérent à la variation des rayons de courbure des condyles fémoraux à l'extrémité inférieure du fémur, lesdits condyles étant en contact avec le tibia dans la cavité glénoïdienne. Ces rayons varient d'avant en arrière entraînant un déplacement également d'avant en arrière des centres instantanés de flexion-rotation du genou au cours de la flexion. Autrement dit, du fait d'une part de la présence des condyles fémoraux avec des rayons de courbure variables, et d'autre part de l'élasticité non physiologique des ligaments synthétiques, une tension isométrique continuelle d'un tel ligament mono-brin ne peut être obtenue.

On a alors proposé de faire appel à deux ligaments mono-brins pour s'affranchir de cet inconvénient rédhibitoire. Le document US - A - 4 411 027 décrit un matériel de suture rotulien, réalisé à partir de deux brins individuels. En fait, il fait appel non pas à un, mais à deux matériels de suture. Ces deux matériels sont mis en place en avant de l'articulation du genou, selon la technique classique dite du "hauban", à savoir, que l'un des matériels de suture fait simplement le tour de deux points d'appui ménagés respectivement sur le tendon quadricipital (extrémité inférieure du muscle antérieur de la cuisse également dénommé quadriceps) et sur le tibia, et décrit un ovale, et que l'autre, également fixé au moyen des deux points d'appui mentionnés ci-dessus, décrit un 8. Cette technique est à ce jour bien connue dans le cadre de la réparation des tendons rotuliens. Toutefois elle se heurte à de notables inconvénients. Tout d'abord, le fait d'utiliser plusieurs matériels de suture provoque, lorsque l'articulation travaille, un coulissement d'un brin par rapport à l'autre, générant de fait des frottements, certes favorables pour la cicatrisation du tendon rotulien, mais qui génèrent une usure prématurée et rédhibitoire des dits brins à travers leur passage dans le tendon et dans le tibia, ne leur permettent en aucun cas d'assurer une rôle prothétique. Ensuite, comme dans le cas précédent, du fait de l'indépenance des deux brins, il n'y a jamais report des contraintes d'un brin sur l'autre, ce qui diminue alors la résistance du brin sollicité, affectant de fait sa durée de vie. De plus, afin de permettre la fixation des deux brins sur l'articulation, il est nécessaire de creuser dans l'os et le tendon concernés des tunnels de taille relativement importante pour permettre le passage des deux brins, provoquant ainsi un affaiblissement de ceux-ci. Enfin, si l'on désirait appliquer les enseignements de ce document dans le cadre du remplacement du ligament croisé antérieur du genou, la non solidarisation des deux brins et le coulissage dans les tunnels d'insertion impliqueraient que lors du travail de l'articulation, une tension permanente de la prothèse ne pourrait être obtenue pour tous les degrés de flexion-rotation. Il est d'ailleurs à noter que ce document revendique non pas une prothèse ligamentaire mais un matériel de suture résorbable dans le cadre de la réparation des tendons rotuliens, donc d'application et de but différents.

La présente invention vise à pallier ces inconvénients. Elle propose une prothèse pour ligament synthétique pour le genou, réalisé en matière biocompatible, comprenant une partie ligamentaire active adaptée à être disposée entre les zones de pénétration intra-articulaire fémorales et tibiales, définissant les entrées de tunnels ménagés respectivement dans le fémur et dans le tibia et de la sortie desquels sortent les extrémités dudit ligament, lesdites extrémités formant attaches en vue d'être fixées respectivement sur le fémur et sur le tibia.

La partie ligamentaire active comprend :

– un premier jeu de deux éléments principaux sensiblement parallèles avant l'implantation, prolongés par les dites attaches, solidarisés entre eux au niveau de la dite zone ligamentaire active,

– ainsi qu'un second jeu de deux éléments intermé diaires croisés au niveau de la dite partie liga mentaire active, et solidaires du dit premier jeu d'éléments principaux à partir et au niveau des zones de pénétration intra-articulaires fémorales et tibiales, en direction de la sortie desdits tunnels.

En d'autres termes, la présente invention propose une prothèse pour ligament synthétique croisé du genou, constituée de deux faisceaux indépendants de fibres synthétiques ou de groupes de fibres, se terminant chacun par une extrémité supérieure condylienne et inférieure glénoïdienne (tibiale) indépendantes les unes des autres, solidarisés à deux groupes de fibres de même nature, reliant entre eux lesdits faisceaux indépendants. Autrement dit, le ligament proposé par la présente invention est du type multi-brins, mais ne constitue qu'une seule entité.

Avantageusement, en pratique :

– les zones d'insertion fémorales des éléments principaux sont distinctes ;

– le ligament est réalisé en matière textile monopièce ;

– les dits premier et second jeux d'éléments sont réalisés en matière textile bio-compatible tricotée et le dit second jeu d'éléments intermédiaires est issu dudit premier jeu d'éléments principaux au voisinage des zones de pénétration intra-articulaires fémorales et tibiales ;

– la zone de solidarisation des éléments principaux au niveau de la partie ligamentaire active est réalisée par un moyen choisi dans le groupe constitué par le tressage, le tricotage, le soudage et la couture ;

– la matière textile biocompatible est un mono ou un multifilament choisi dans le groupe constitué par les polyesters, le polypropylène et le verre ;

– les éléments intermédiaires sont solidarisés aux éléments principaux par soudage, notamment par thermosoudage ;

– les extrémités du ligament formant attaches, destinées à être fixées sur le tibia et sur le fémur sont revêtues d'une gaine en polyéthylène munie d'un chas, destinée à permettre le passage desdites extrémités dans les dits tunnels.

La manière dont l'invention peut être réalisée et les avantages qui en découlent ressortiront mieux de l'exemple de réalisation qui suit, donné à titre indicatif et non limitatif, à l'appui des figures annexées.

La figure 1 est une représentation schématique plane d'une prothèse conforme à l'invention.

La figure 2 est une représentation schématique de profil de l'articulation du genou droit, dont le condyle interne a été enlevé, et mettant en évidence les orifices intra-articulaires fémoraux et tibiaux de fixation de la prothèse, ménagés dans les surfaces d'insertion anatomiques du ligament croisé antérieur.

La figure 3 est une représentation schématique de profil, de l'articulation en extension complète d'un genou droit, dont le condyle interne à été enlevé, sur laquelle la prothèse conforme à l'invention est en place.

La figure 4 est une vue analogue à la figure 3, mais dans laquelle l'articulation est en flexion.

La figure 5 est une vue analogue aux figures 3 et 4, dans laquelle l'articulation est en position intermédiaire par rapport à celles desdites figures 3 et 4.

La figure 6 est une vue explicative très schématique dans laquelle on a représenté la prothèse en place sur l'articulation, cette dernière étant vue de face. Cette figure est purement schématique et n'a d'autres buts que de favoriser la compréhension .

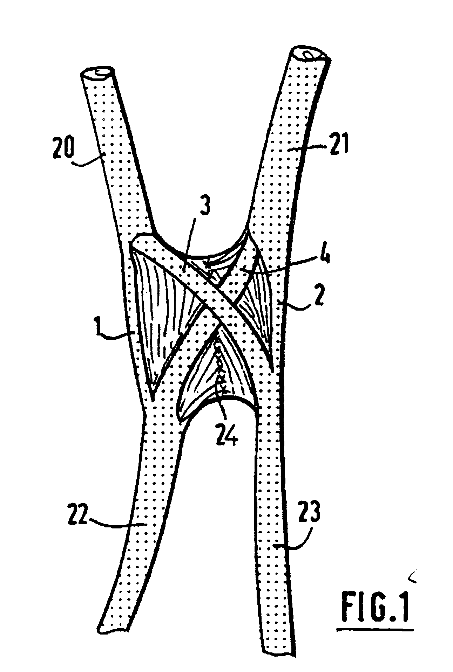

Comme on peut le voir au sein de la figure 1, la prothèse du ligament croisé antérieur conforme à l'invention se présente sous la forme de deux éléments principaux (1) et (2), respectivement le faisceau postéro-externe (1) et le faisceau antéro-interne (2).

Ces deux éléments principaux (1,2) ont une longueur comprise entre quatorze et vingt-trois centimètres. Ils présentent chacun une extrémité fémorale, respectivement (20) et (21) et une extrémité tibiale, respectivement (22) et (23), jouant le rôle d'attaches en vue de la fixation du ligament respectivement sur le fémur (7) et sur le tibia (8). Ils ont un diamètre typique de l'ordre de cinq à six millimètres, mais il va de soi que ce dernier peut être adapté à la morphologie ou à l'activité du receveur de la prothèse.

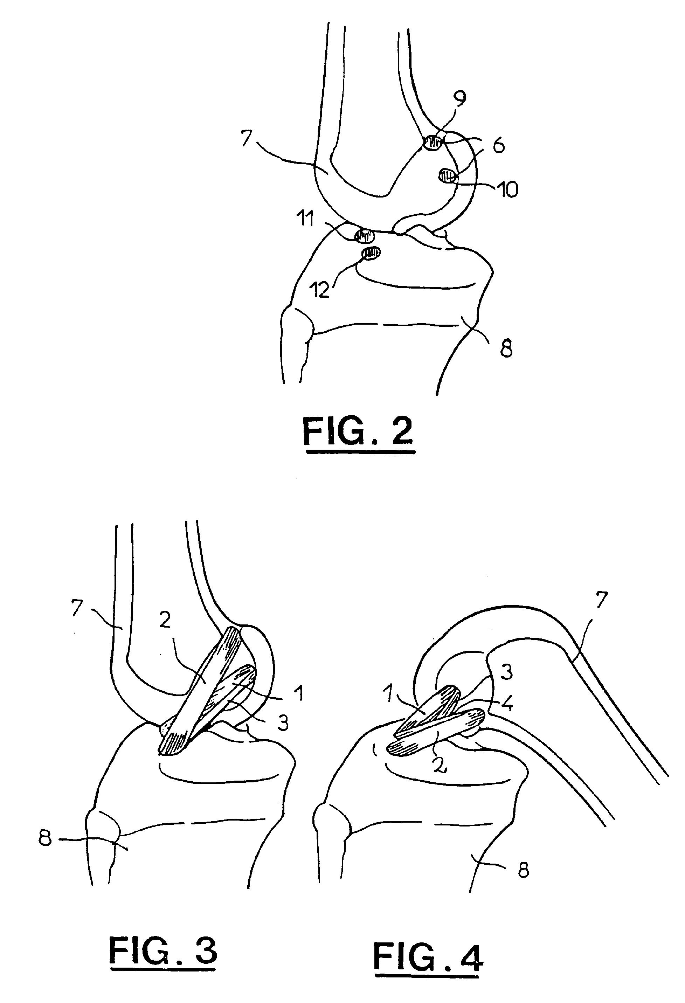

Bien qu'apparaissant parallèles sur la figure, ces deux éléments ne sont pas parallèles lorsqu'ils sont fixés en place au sein de l'articulation. En effet, comme on peut le voir sur les figures 2 à 5, lorsque la prothèse est en place sur l'articulation, elle est légèrement torsadée, inhérent d'une part à l'anatomie de l'articulation, et d'autres part aux zones d'insertion ménagées dans celle-ci.

De manière avantageuse, ces faisceaux se présentent sous la forme de tricots tubulaires multi-filaments de matériau textile biocompatible, notamment en polyester.

Il est bien entendu que ces éléments pourraient être réalisés sous forme de tresse, voire même de tricots tubulaires mono-filament sans se départir de l'esprit de l'invention.

De plus, afin de protéger les faisceaux, et afin de leur donner un effet élastique, ils sont avantageusement enduits d'une couche de polyuréthane biocompatible au niveau de leur champ d'action dans l'articulation, c'est à dire au niveau de la partie ligamentaire active, et également, au niveau du début des extrémités (20,21, 22,23) insérées dans les tunnels ménagés dans le fémur et dans le tibia, en vue de la fixation des dites attaches sur le fémur (7) et sur le tibia (8).

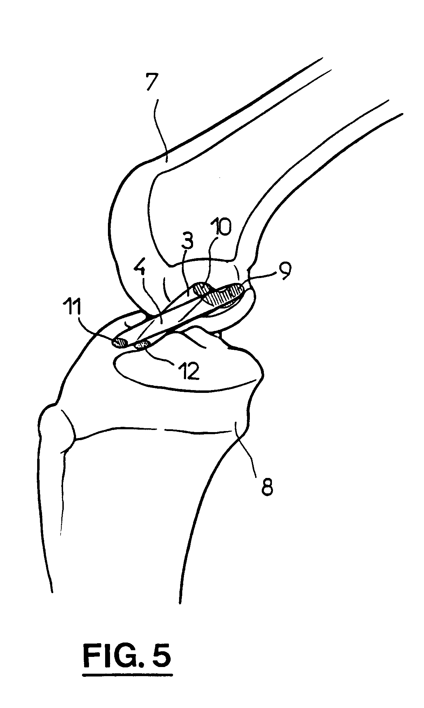

L'insertion de ces deux éléments (1) et (2) est effectuée au niveau des surfaces d'insertion du ligament croisé antérieur naturel à la remarque près que les deux insertions supérieures, c'est-à-dire fémorales, sont distinctes. Elles ont lieu au niveau de la berge interne postérieure du condyle externe du fémur. On a représenté en zone hachurée dans la figure 5 le lieu de la surface d'insertion fémorale du ligament anatomique.

L'insertion inférieure des deux éléments (1) et (2) s'effectue également en des lieux distincts au niveau de la surface pré-spinale antérieure du tibia (8).

Selon une caractéristique importante de l'invention, les deux éléments principaux (1) et (2) sont solidarisés au niveau (24) de la partie ligamentaire active, notamment par tricotage. Il va de soi qu'ils peuvent l'être par tout autre moyen approprié, tel que par exemple le tressage, le soudage ou la couture.

De plus, selon une autre caractéristique fondamentale de la présente invention, le ligament comporte également un jeu d'éléments intermédiaires (3,4), également réalisés en matériau textile biocompatible. Ils ont un diamètre typique compris entre deux et trois millimètres.

Ces éléments (3,4) sont directement issus des ligaments principaux (1,2), au niveau des zones de pénétration intra-articulaires (9,10,11,12), et sont de fait solidarisés aux dits éléments principaux (1,2). Ces éléments (3,4) sont croisés au niveau de la partie ligamentaire active. En d'autres termes,le ligament intermédiaire (3), issu de l'élément principal postéro-externe (1) au niveau de la zone de pénétration intra-articulaire fémorale (10), se réintègre dans l'élément principal antéro-interne (2) au niveau de la zone de pénétration intra-articulaire tibiale (12). De même, le ligament intermédiaire (4), issu du ligament principal antéro- interne (2) au niveau de la zone intra-articulaire fémorale (9), se réintègre dans l'élément principal postéro-externe (1) au niveau de la zone de pénétration intra-articulaire tibiale (11).

De fait, le ligament conforme à l'invention est réalisé en matière textile monopièce. De la sorte il y a toujours solidarisation des éléments intermédiaires avec les éléments principaux, notamment au niveau et à partir des zones de pénétrations intra-articulaires fémorales et tibiales en direction de la sortie des tunnels d'insertion et de fixation dudit ligament. De même, il y a également solidarisation des éléments principaux au niveau de la partie ligamentaire active.

Dans une autre version de la présente invention, les éléments intermédiaires sont également solidarisés aux éléments principaux au niveau et à partir des zones de pénétration intra-articulaires fémorales et tibiales en direction de la sortie des tunnels d'insertion et de fixation, sans toutefois faire partie intégrante des dits éléments principaux, mais par soudage ou par thermosoudage.

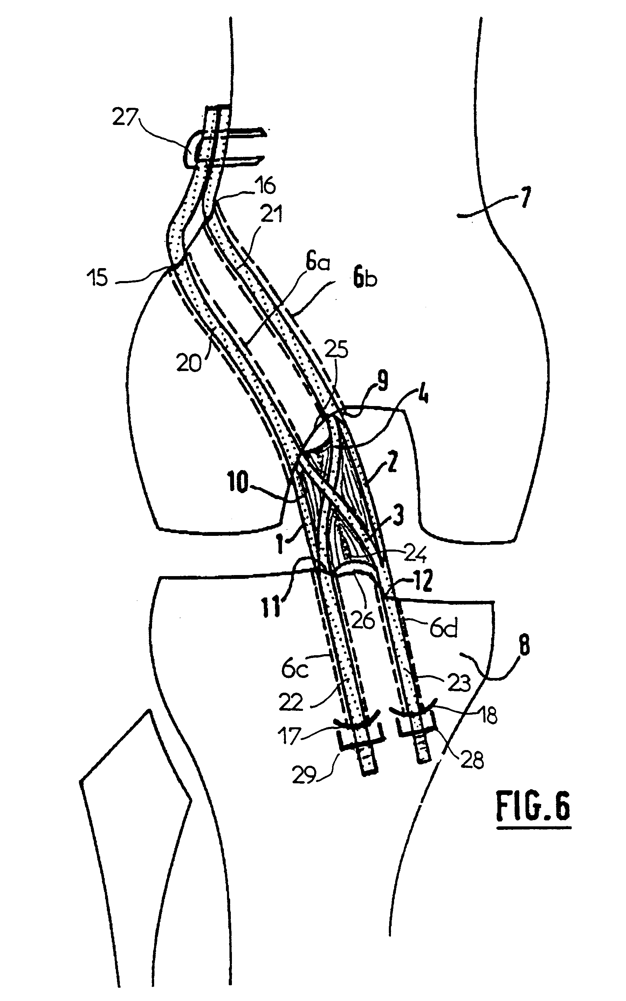

La fixation de la prothèse s'effectue comme suit (voir figure 6). On perce, au préalable et par tout moyen approprié, notamment au moyen de mèches, dans le fémur (7) et dans le tibia (8) au niveau des zones d'insertion du ligament naturel, des tunnels d'insertion fémoraux (6a,6b) et tibiaux (6c,6d),dont on a représenté les orifices intra-articulaires respectivement fémoraux (9,10) et tibiaux (11,12) au sein de la figure 2. Les zones d'insertion extra-articulaires fémorales et tibiales des dits tunnels sont représentées sous les références respectives (15,16) et (17,18) au sein de la figure 6. Pour la réalisation de ces tunnels, l'articulation du genou est en flexion voisine de 60 - 70 degrés. Ces tunnels (6) ont typiquement un diamètre de cinq à six millimètres. Les extrémités des éléments principaux (1,2) sont insérées dans ces tunnels (6) au moyen des attaches (20,21,22, 23), recouvertes d'une gaine (non représentée) réalisée en polyester, et munie d'un chas, pour rendre plus aisé cette insertion. Les extrémités fémorales (20,21) sont tout d'abord tirées vers le haut, afin de mettre en contact la zone de solidarisation (24) des deux éléments principaux (1) et (2) avec la zone (25) située entre les deux tunnels (6) d'insertion fémorale, puis torsadées l'une avec l'autre, et enfin fixées sur le fémur (7) au moyen d'une agrafe (27) d'usage courant pour cette application.

On procède alors à la fixation des attaches tibiales (22,23). On fait tout d'abord affleurer la zone de solidarisation (24) des deux éléments principaux (1,2) avec la zone (26) située entre les deux zones de pénétration intra-articulaires tibiales (11,12). Puis on insert dans les tunnels (6c,6d) correspondants les dites attaches, également recouvertes d'une gaine rigide en polyéthylène, munie d'un chas. Puis on applique une tension de l'ordre de dix kilogrammes sur l'élément antéro-interne (2), l'articulation du genou étant en extension, avant de fixer au moyen d'une agrafe (28) le dit élément sur le tibia. De même, on applique une tension du même ordre sur l'élément postéro-externe (1), l'articulation du genou étant en flexion d'un angle de 30 degrés, avant de le fixer, également au moyen d'une agrafe (29) sur le tibia (8).

On a représenté dans les figures 3 à 5, le ligament conforme à l'invention en place sur l'articulation. Dans la figure 3, seul l'élément intermédiaire (3) est visible, l'autre élément intermédiaire (4) étant caché par l'élément antéro-interne (2). Dans la figure 4, les deux éléments intermédiaires (3,4) ainsi que les deux éléments principaux (1,2) sont visibles, l'articulation se trouvant en flexion complète. On n'a représenté dans la figure 5 que les éléments intermédiaires (3,4), afin de mieux mettre en évidence leur positionnement dans le cadre de l'architecture et la mise en place du ligament conforme à l'invention.

De la description précédente, il ressort que du fait des variations des rayons de courbure des condyles fémoraux, et de par le mode de mise en place du ligament conforme à l'invention, il existe en permanence une partie du ligament sous tension, limitant voire annulant les mouvements de tiroir antérieur du tibia sous le fémur.

Au cours de la flexion représentée dans la figure 4, du fait des rayons de courbure variables des condyles fémoraux, la tension des filaments constitutifs de la prothèse ainsi réalisée est progressivement transférée de la partie antérieure desdits filaments (tendus lors de l'extension) à la partie postérieure, en incluant l'entrecroisement des filaments des éléments intermédiaires. En d'autres termes, il existe toujours une partie des filaments constitutifs des éléments de la prothèse tendue.

De même, en cours de la rotation, les deux faisceaux en rotation interne vont avoir tendance à se tendre le long de leur axe, d'où une diminution de la distance séparant les condyles du fémur à la cavité glénoïdienne, provoquant une coaptation des surfaces articulaires.

En rotation externe, les deux éléments principaux (1) et (2) vont avoir tendance à se détendre, et les éléments intermédiaires limitent, de par leur direction, la tendance au tiroir antérieur et à la rotation externe.

Ainsi, cette disposition géométrique permet d'obtenir à chaque degré de flexion la tension d'un groupe de fibres, la zone mise en tension de ce montage variant au cours de la flexion.

En outre, lors de contraintes excessives, comme par exemple lors d'efforts sportifs, c'est à dire lorsque la tendance aux mouvements de tiroir antérieur augmente, une quantité supérieure de fibres constitutives des différents éléments du ligament conforme à l'invention est sollicitée pour lutter et s'opposer à ces mouvements de tiroir antérieur. Cette caractéristique est inhérente à la solidarisation des différents faisceaux entre eux, augmentant la résistance mécanique du ligament.

Ainsi, de la présente invention, se dégagent un certain nombre d'avantages que les prothèses de ligament croisé connues à ce jour ne permettaient pas d'obtenir, notamment l'augmentation de l'efficacité du ligament dans le contrôle du tiroir antérieur et de la coaptation articulaire, ce qui était absolument impossible d'obtenir avec des ligaments à un seul faisceau, ou avec des prothèses multi-ligaments.

## Revendications

1. Ligament synthétique pour le genou, réalisé en matière biocompatible, comprenant une partie ligamentaire active adaptée à être disposée entre les zones de pénétration intra-articulaire fémorales (9,10) et tibiales (11,12), définissant les entrées de tunnels (6a,6b,6c,6d) ménagés respectivement dans le fémur (7) et dans le tibia (8) et de la sortie desquels sortent les extrémités dudit ligament, lesdites extrémites formant attaches (20,21,22,23) en vue d'être fixées respectivement sur le fémur (7) et sur le tibia (8), ladite partie ligamentaire active comprenant un premier jeu de deux éléments principaux (1,2) sensiblement parallèles avant l'implantation, prolongés par les dites attaches (20,21,22,23), solidarisés entre eux au niveau (24) de la dite zone ligamentaire active, **caractérisé** en ce que ladite partie ligamentaire active comprend en plus un second jeu de deux éléments intermédiaires (3,4) croisés au niveau de la dite partie ligamentaire active, et solidaires du dit premier jeu d'éléments principaux (1,2) à partir et au niveau des zones de pénétration intra-articulaires fémorales (9,10) et tibiales (11,12), en direction de la sortie desdits tunnels (6).

2. Ligament synthétique pour le genou selon la revendication 1, caractérisé en ce qu'il est réalisé en matière textile monopièce.

3. Ligament synthétique pour le genou selon l'une des revendications 1 et 2 caractérisé en ce que les dits premier (1,2) et second (3,4) jeux d'éléments sont réalises en matière textile bio-compatible tricotée et en ce que le dit second jeu d'elements intermédiaires (3,4) est issu dudit premier jeu d'éléments principaux (1,2) au voisinage des zones de pénétration intra-articulaires fémorales (9,10) et tibiales (11,12).

4. Ligament synthétique pour le genou selon l'une des revendication 1 à 3, caractérisé en ce que la zone de solidarisation (24) des éléments principaux (1,2) au niveau de la partie ligamentaire active est réalisée par un moyen choisi dans le groupe constitué par le tressage, le tricotage, le soudage et la couture.

5. Ligament synthétique pour le genou selon l'une des revendications 1 à 4, caractérisé en ce que la matière textile biocompatible est un mono ou un multifilament choisi dans le groupe constitue par les polyesters, le polyropylène et le verre.

6. Ligament synthétique pour le genou selon la revendication 1, caractérisé en ce que les elements intermédiaires (3,4) sont solidarises aux elements principaux (1,2) par soudage, notamment par thermosoudage.

7. Ligament synthétique pour le genou selon l'une des revendications 1 à 6, caractérisé en ce que les extrémités du ligament formant attaches (20,21,22, 23), destinées à être fixées sur le tibia (8) et sur le fémur (7) sont revêtues d'une gaine en polyéthylène munie d'un chas, destinée à permettre le passage desdites extrémités dans les dits tunnels (6).

**Patentansprüche**

1. Synthetisches Kniegelenkband aus biologisch verträglichem Material, mit einem aktiven Bandabschnitt, der dazu vorgesehen ist, zwischen den femoralen (9, 10) und tibialen (11, 12) Gelenkeindringstellen zum Liegen zu kommen, wobei letztere Einlässe von Kanälen (6a, 6b, 6c, 6d) umgrenzen, die jeweils im Femur (7) und in der Tibia (8) ausgespart sind, und aus deren Auslässe die äußeren Enden des Bandes austreten, wobei die äußeren Enden sichtbare Befestigungselemente (20, 21, 22, 23) bilden, die am Femur (7) bzw. an der Tibia (8) befestigt sind, wobei der aktive Bandabschnitt eine erste Gruppe an zwei Hauptelementen (1, 2) aufweist, die vor der Implantation etwa parallel zueinander verlaufen, die ferner durch die Befestigungselemente (20, 21, 22, 23) verlängert sind, und die auf Höhe (24) des aktiven Bandabschnittes untereinander verbunden sind, **dadurch gekennzeichnet**, daß der aktive Bandabschnitt darüberhinaus eine zweite Gruppe an zwei, auf Höhe des aktiven Bandabschnittes gekreuzt verlaufenden zwischenliegenden Elementen (3, 4) aufweist, die, ausgehend von und auf Höhe der femoralen (9, 10) und tibialen (11, 12) Gelenkeindringstellen, in Richtung der Auslässe der Kanäle (6), zusammenhängend mit der ersten Gruppe an Hauptelementen (1, 2) sind.

2. Synthetisches Kniegelenkband nach Anspruch 1, dadurch gekennzeichnet, daß es aus einstückigem textilen Material hergestellt ist.

3. Synthetisches Kniegelenkband nach Anspruch 1 oder 2, dadurch gekennzeichnet, daß die erste (1, 2) und zweite (3, 4) Gruppe an Elementen aus gestricktem bzw. gewirktem, biologisch verträglichem Textilmaterial hergestellt sind und daß, benachbart zu den femoralen (9, 10) und tibialen (11, 12) Gelenkeindringstellenbereichen, die zweite Gruppe an zwischenliegenden Elementen (3, 4) aus der ersten Gruppe an Hauptelementen (1, 2) hervorgeht.

4. Synthetisches Kniegelenkband nach einem der Ansprüche 1 bis 3, dadurch gekennzeichnet, daß der Verbindungsbereich (24) der Hauptelemente (1, 2) auf Höhe des aktiven Bandabschnittes untereinander durch Maßnahmen hergestellt ist, die ausgewählt sind, aus der Gruppe bestehend aus Flechten, Stricken, Verschweißen und Nähen.

5. Synthetisches Kniegelenkband nach einem der Ansprüche 1 bis 4, dadurch gekennzeichnet, daß das biologisch verträgliche textile Material ein Mono- oder Multifilament ausgewählt aus der Gruppe bestehend aus Polyester, Poly-propylen und Glas ist.

6. Synthetisches Kniegelenkband nach Anspruch 1, dadurch gekennzeichnet, daß die zwischenliegenden Elemente (3, 4) durch Verschweißen, insbesondere durch Wärmeverschweißen mit den Hauptelementen (1, 2) verbunden sind.

7. Synthetisches Kniegelenkband nach einem der Ansprüche 1 bis 6, dadurch gekennzeichnet, daß die äußeren Enden des Bandes, die die Befestigungselemente (20, 21, 22, 23) bilden, und die dazu bestimmt sind, an der Tibia (8) und an dem Femur (7) befestigt zu werden, mit einer ein Öhr aufweisenden Umhüllung aus Polyethylen überzogen sind, um den Durchtritt der äußeren Enden durch die Kanäle (6) zu ermöglichen.

**Claims**

1. A biocompatible synthetic ligament for the knee, comprising an active part designed to be placed between the intra-articular femoral (9,10) and tibial (11,12) penetration areas, defining the entrances of tunnels (6a,6b,6c,6d) made in the femur (7) and in the tibia (8), respectively, and from the exit of which emerge the ends of said ligament, said ends forming ties (20,21,22,23) to be fixed to the femur (7) and to the tibia (8), respectively, said active part of the ligament comprising a first set of two principal elements (1,2) that are substantially parallel before implantation, extended by said ties (20,21,22,23), integral with each other at the level (24) of said active part of the ligament, wherein said active part of the ligament further comprises a second set of two intermediate elements (3,4) that are crossed at the level of said active part of the ligament and integral with first set of principal elements (1,2) starting from, and at the level of, the intra-articular femoral (9,10) and tibial (11,12) penetration areas, in the direction of the exit of said tunnels (6).

2. The synthetic ligament for the knee according to claim 1, wherein it is made of a one-piece textile material.

3. The synthetic ligament for the knee according to either claims 1 and 2, wherein said first (1,2) and second (3,4) sets of elements are made of a biocompatible textile material, and wherein said second set of intermediate elements (3,4) emerges from said first set of principal elements (1,2) in the vicinity of the intra-articular femoral (9,10) and tibial (11,12) penetration areas.

4. The synthetic ligament for the knee according to any one of the claims 1 to 3, wherein the interlocking area (24) of the principal element (1,2) at the level of the active part of the ligament is constructed by means selected from the group established by braiding, knitting, welding and sewing.

5. The synthetic ligament for the knee according to any one of the claims 1 to 4, wherein the biocompatible textile material is a monofilamentous or multifilamentous material selected from the group comprised of polyesters, polypropylène and glass.

6. The synthetic ligament for the knee according to claim 1, wherein the intermediate elements (3,4) are made integral with the principal elements (1,2) by welding, in particular by heat sealing.

7. The synthetic ligament for the knee according to any one of the claims 1 to 6, wherein the ends of the ligament forming ties (20,21,22,23) designed to be attached to the tibia (8) and to the femur (7), are covered with a polyethylene sheath provided with a needle eye aperture to enable passage of said ends in said tunnels (6).

FIG.1

**FIG. 2**

**FIG. 3**

**FIG. 4**

FIG. 5

**FIG.6**